(19) Europäisches Patentamt
European Patent Office
Office européen des brevets

(11) Publication number: 0 294 216
A2

(12) **EUROPEAN PATENT APPLICATION**

(21) Application number: 88305083.3

(22) Date of filing: 03.06.88

(51) Int. Cl.⁴: **G 01 N 33/50**
**C 12 Q 1/34**

(30) Priority: 04.11.87 US 117168  05.06.87 US 58347
23.05.88 US 197471

(43) Date of publication of application:
07.12.88 Bulletin 88/49

(84) Designated Contracting States:
AT BE CH DE ES FR GB GR IT LI LU NL SE

(71) Applicant: INTERFERON SCIENCES, INC.
783 Jersey Avenue
New Brunswick New Jersey 08901 (US)

(72) Inventor: Khavkin, Theodor
126 Montgomery Street
Highland Park New Jersey 08904 (US)

Kuchler, Martha
11 Dogwood Court
East Brunswick New Jersey 08816 (US)

Liao, Mei-June
37 Norton Road
Monmouth Junction New Jersey 08852 (US)

(74) Representative: Hale, Stephen Geoffrey et al
J.Y. & G.W. Johnson Furnival House 14/18 High Holborn
London WC1V 6DE (GB)

(54) A functional assay for T-Lymphocytes.

(57) The presence of activated or pre-sensitized T-lymphocytes in whole blood, or in a mixture of isolated leukocytes, is detected by means of forming autologous E-rosettes and mixed leukocyte-erythrocyte aggregates which are stable at 37°C. More specifically, by means of stimulating the cells using either a non-erythroagglutinating mitogen or an antigen, and removing sialic acid with, for example, neuraminidase, stable E-rosettes and mixed leukocyte-erythrocyte aggregates may be formed and counted. Also provided are kits for use in assaying for the presence of mitogen-activated or antigen pre-sensitized T-lymphocytes, without using sheep red blood cells, immunostaining or radioactive isotopes.

EP 0 294 216 A2

**Description**

## A FUNCTIONAL ASSAY FOR T-LYMPHOCYTES

TECHNICAL FIELD OF INVENTION

This invention relates to a method for forming stable autologous E-rosettes ("SAR") in cultures of mitogen or antigen stimulated human blood cells. In one aspect, this invention relates to the formation of SAR and mixed aggregates of erythrocytes and leukocytes, by T cells activated with a non-erythroagglutinating mitogen, or by sensitized T cells which are stimulated with the sensitizing antigen, provided that contacts between T cells and human red blood cells are enhanced by the removal of sialic acid residues from either the T cell or erythrocyte membranes, or from both. In another aspect, this invention relates to an assay for activated or antigen-sensitized T-lymphocytes based upon the involvement of autologous erythrocytes in the stable E-rosette formation and leukocyte aggregation observed upon stimulation with non-erythroagglutinating mitogens or antigen, respectively.

BACKGROUND OF THE INVENTION

Human T cells all known to form E-rosettes in vitro with red blood cells (RBC) from many species including autologous red blood cells [M. Amiot et al., in Leukocyte Typing, p. 281 (1984); V. Nalet and C. Fournier, Cell. Immunol., 96, pp. 126-36 (1985)]. This phenomenon is the basis of an immunological assay for, among other things, the defining of T cells [W. H. West, E-Rosette Formation in Immunodiagnosis", Immunodiagnosis of Cancer, pp. 704-721 (1979)]. Typically, sheep red blood cells (SRBC) are used to form E-rosettes because sheep erythrocytes show greater reactivity with the CD2 surface antigen of T-lymphocytes than do human RBC. The CD2 antigen has been shown to mediate the RBC attachment to T cells [A. Bernard et al., Leukocyte Typing II, 1, pp. 53-66, (1986)]. This common laboratory assay for resting T-lymphocytes cells requires temperatures of between 5 and 24°C [See generally, W. H. West, supra].

SRBC form rosettes with resting T cells, as well as mitogen stimulated T cells. Furthermore, SRBC can form giant rosettes, which are stable at 37°C temperature, with activated T cells (mitogen stimulated) or with resting T cells when neuraminidase or AET is used to pre-treat either the SRBC or the resting T cells [U. Galili and M. Schlesinger, J. Immunol., 112, p. 1628 (1974); J. P. van Wauwe and J. Goosens, Cell. Immunol., 68, p. 181 (1982)]. The increased expression of CD2 surface antigen on activated T cells largely accounts for the proportional increase in the number of these giant stable rosettes [S. K. Oh et al., Clin.Immunol.Immunopathol, 38, p. 55 (1968)].

While the routine assay for T-lymphocytes using E-rosettes remains a satisfactory one for laboratory use, it involves the laborius procedure of having first to isolate lymphocytes, and the additional expense of obtaining sheep RBC. The conventional production of stable E-rosettes requires the same procedures and means. Accordingly, efforts have been made to develop a simpler and less expensive assay for T cell presence using human RBC.

Human RBC form rosettes with resting autologous T cells, at a much lower rate than do SRBC. These rosettes are generally fragile and depend upon the concentration of proteins in the culture media. Furthermore, the routine assay for forming autologous E-rosettes, requires refrigerator temperatures of 4°C even when activated or neuraminidase-treated cells are used [L. A. Gallinger et al., Int. J. Cancer, 26, pp. 139-50 (1980); C. Fournier et al., Human Immunol., 16, p. 81 (1986)].

It is known that T cell activation by mitogens or antigens in vitro involves the formation of leukocytic collections (aggregates) which are largely composed of lymphocytes and monocytes. This leukocyte aggregation represents an immunologic event, and is mediated by appropriate active substances - lymphokines and monokines [P. Lipsky, A. Rosenthal, J. Exp. Med., 141: 138-153; 0. Werdelin in Macrophage Regulation of Immunity, pp. 213-229, (1980)]. In leukocyte cultures which have been stimulated with some mitogenic lectins such as phytohemagglutinins P and E (PHAP, PHAE) in the presence of autologous RBC, the RBC attach to aggregated leukocytes resulting in formaticn of "mixed agglutinates" (mixed aggregates) [S. Yachnin et al., Cell Immunol., 72, pp. 569-589, (1972)]. The RBC attachment to T cells in such mixed aggregates is different from that observed with E-rosettes; it generally represents a chemical rather than immunologic event, because PHAP and PHAE demonstrate chemical affinity to the membrane structures on both RBC and lymphocytes [J. Egorin, et al., J. Biol. Chem., 254, pp. 894-898 (1979)]. The PHAP or PHAE induced mixed RBC-leukocyte aggregation is also accompanied by the RBC attachment to other RBC. This phenomenon is well known as a lectin-induced RBC agglutination.

Except for the publications of T. Khavkin et al., there have been no reports in the literature that human RBC can form stable rosettes with autologous T cells. In J. Leukocyte Biol., 40, N3 Abstract 165 (1986), T. Khavkin et al. have reported the formation of autologcus stable E-rosettes around activated lymphocytes in phytohemagglutininexposed cultures of human leukocytes and red blood cells at temperatures of 37°C. However, these stable rosettes were primarily formed within mixed collections composed of agglutinated red blood cells and aggregated leukocytes. Moreover, the induction of stable autologous rosettes by phytohemagglutinins P and E described by T. Khavkin et al. is acccmpanied by nonspecific agglutination of red blood cells caused by these lectins, which would interfere with the assessment of the extent of the immunological event (E-rosette formation).

Improved methods for assaying for T cells through the formation of SAR and leukocyte aggregates using human RBC remains desirable for many reasons. In particular, methods are needed to assay for the presence of activated T cells or pre-sensitized T cells with simpler, safer and less expensive tools than those which are the presently in use.

## DISCLOSURE OF THE INVENTION

The present invention provides a method for assaying for the presence of either activated or pre-sensitized human T-lymphocytes in whole blood comprising the formation of stable E-rosettes and mixed RBC-leukocyte aggregates in culture, using autologous erythrocytes. In accordance with this invention, the activation (i.e. blast transformation) of T cells with a non-erythroagglutinating mitogen and the removal of sialic acid residues from either the T cells, the red blood cells or both, results in the formation of giant E-rosettes around the activated T cells, which are stable at 37°C. Alternatively, pre-sensitized T cells can be detected by using the sensitizing antigen in this assay. In addition, this method provides for the formation of leukocyte aggregates with erythrocyte involvement (mixed aggregates) upon stimulation with non-erythroagglutinating mitogens. The present method is particularly useful in assays for evaluating T cell response to mitogenic or antigenic stimulation, without using radioactive isotopes, sheep red blood cells or immuno-staining. In addition, the present invention provides a means for detecting activated T cells which have been previously sensitized with various antigens including those of pathogenic infectious agents. For the purposes of this application, the formation of both E-rosettes and mixed leukocyte-erythrocyte aggregates ("MA") are significant for the present assay for T-lymphocytes.

## BEST MODE OF THE INVENTION

In order that the invention herein described may be more fully understood, the following detailed description is set forth.

In the description the following terms are employed:

T-lymphocytes (T cell) - a population of lymphocytes serving as effector cells in cell-mediated immunity, and regulator cells (helper and suppressors) in antibody production by B-lymphocytes. A decrease in the number of T-lymphocytes or their functional suppression in the human body may result in an immunodeficiency.

T-lymphocyte activation - augmentation of the level of T cell functions that is usually associated with an increased expression and rearrangement of lymphocyte surface antigens, and with lymphocyte blast transformation (activation, proliferation). The increased consumption of thymidine by activated T cells is utilized in the typical assay for T cell activation and responsiveness (Council on Scientific Affairs of the American Medical Association, "In vitro testing for allergy. Report II of the Allergy Panel". J. Amer. Med. Ass. 258:1639-1643, 1987).

Mitogens - Various substances, regarding chemical or molecular composition, which are capable of provoking in vitro T-cell activation. Examples of mitogens are bacterial products such as staphylococcal enterotoxin A (SEA), antilymphocyte immunoglobulins such as OKT3 monoclonal antibody, and plant substances - termed "mitogenic lectins" - such as phytohemagglutinin P (PHAP) and its isolectins (PHAE and PHAL), and concanavalin A (Con A). Along with their mitogenic activity, mitogenic lectins agglutinate all types of blood cells. PHAP and PHAE agglutinate both red and white blood cells. PHAL and Con A (at mitogenically optimal concentrations) have only leukoagglutinating capacity, i.e. negligible erythroagglutinating capacity. Unlike lectins, SEA and OKT3 possess neither erythro- nor leukoagglutinating capacities.

Antigens - substances capable of inducing humoral and cell mediated immune responses; for example: a) production of an antibody by B cells, which is capable of reacting specifically with an antigen; (b) appearance of T lymphocytes specifically sensitized to this antigen and capable of responding to it by blast transformation. Examples of antigens are antigens of infectious agents, such as viruses, rickettsiae, bacteria, and protozoa as well as various proteins, glyco- and lipoproteins.

Leukocyte aggregation - an immunologic event manifested by formation of cellular aggregates consisted largely of lymphocytes and monocytes/macrophages. The aggregation occurs in leukocyte cultures upon mitogenic or antigenic stimulation. Leukocyte aggregation provides physical cell-cell contacts that are required for the exchange with an appropriate information between lymphocytes and monocytes/macrophages in the course of activation [R. Bolhuis, Clinics in Immunology and Allergy, 6, pp. 29-90, (1986)].

E-rosette - structure formed by adherence of erythrocytes (red blood cells) around a lymphocyte. SRBC form E-rosettes with human T cells spontaneously at the temperatures ranging from 4 to 24°C: at 37°C these E-rosettes normally dissociate.

Autologous E-rosettes - E-rosettes formed by human red blood cells around autologous T-lymphocytes.

Stable E-rosettes - E-rosette that is stable at 37°C. Stable E-rosettes are formed by SRBC around activated human T-lymphocytes or around resting lymphocytes after the lymphocytes or red blood cells have been treated with neuraminidase or a similar substance.

Neuraminidase (sialidase) - a group of enzymes produced by some viruses, bacteria (as for instance Vibrio cholerae), protozoa, and vertebrates having the common feature of hydrolysing the acetilated neuraminic acids (sialic acids) which constitute integral parts of the cell membrane glycocompounds [See R. Drzeniek, "Viral and Bacterial Neuraminidases," Curr. Topics in Microbiol. Immunol., 59, pp. 35-74, (1972)]. Neuraminidases are capable of splitting sialic acid residues from cell membranes of red blood cells and lymphocytes.

AET - A sulfhydryl reagent: 2-aminoethyisothiouronioum bromide which is capable of removal of sialic acid residues from red blood cell surfaces, thus facilitating the RBC attachment to T lymphocytes [M.E. Kaplan, C. Clark, J.Immunol. Methods, 5, pp. 131-35 (1974)]. AET is currently applied, along with neuraminidase, in the present E-rosetting assay for T-lymphocytes.

This invention provides a simple, efficient and inexpensive method for forming stable autologous E-rosettes using whole blood. More particularly, this method provides an assay for detection of stimulated T cells, upon exposure to a non-erythroagglutinating mitogen, and the simultaneous removal of sialic acid residues from both T cell and red blood cell membranes, with neuraminidase, in order to form both stable E-rosettes and mixed aggregates. Additionally, an assay for the detection of sensitized T cells is provided, using the sensitizing antigen instead of the mitogen in the assay described above. In a preferred embodiment of this invention, a mixture of either the mitogen or antigen is added with neuraminidase to whole blood in vitro, and incubated at 37° in the presence of 5% $CO_2$ for 48 to 96 hours. Alternatively, a mixture of isolated leukocytes and red blood cells may be substituted for the whole blood, provided that neuraminidase is present in the culture medium or that red blood cells have been pretreated with neuraminidase or with 2-Amino-ethyisothiour bromide (hereinafter "AET"). The T cells are preferably stimulated by a mitogen, such as SEA, OKT 3 monoclonal antibody, Con A or PHAL. Antigens such as viral, rickettsial, bacterial, and protozoal antigens can be used in the methods of this invention in order to detect and to evaluate cell-mediated immunity to the respective infectious agent [See L. Bourgeois et al., Infection and Immunity, 27, pp. 483-89 (1980)].

Our studies have shown that in leukocyte cultures stimulated with non-erythroagglutinating mitogens in the presence of neuraminidase, the formation of stable E-rosettes is accompanied by combined erythrocyte-leukocyte aggregation ("MA"). Such mixed aggregation is not observed in cultures stimulated with these mitogens or antigens without the use of neuraminidase, for example. Thus, for the purposes of the present invention, this event is to be considered as an equivalent to the stable autologous E-rosettes. Combined aggregates should be scored together with E-rosettes. We have named these structures "rosette-like patterns". The stable autologous E-rosettes and combined erythrocyte leukocyte aggregates which are formed according to the method of this invention can be conventionally evaluated with a microscope and scored using a hemocytometer.

The present invention provides a particularly efficient method for monitoring the relative number of activated T lymphocytes in evaluating the response of a patient to immunosuppressive drug treatment following transplant surgery. In addition, it provides a simple assay for both the screening of healty individuals for T cell responsiveness, and for the clinical immunodiagnosis of malignant disease; for example, this invention allows for a single-step determination of an expression of cell mediated immunity, including identifying a deficiency in T cells caused by viral infection, such as that caused by AIDS. Further, when a viral, rickettsial or bacterial antigen is used instead of mitogens to activate T cells, the presence or absence of cell-mediated immunity against the respective antigen, and its degree can be measured without the use of radioactive thymidine, according to the methods of this invention. This is particularly useful in assays for determining the presence of previously sensitized T cells to pathogenic agents such as the HIV virus.

In order that the invention described herein may be more fully understood, the following examples are set forth. It should be understood that these examples are for illustrative purposes only, and are not to be construed as limiting this invention in any manner.

## EXAMPLE I

### Whole Blood Assay

We obtained fresh whole blood by venipuncture, and placed it into a tube containing heparin (50 U/ml). We determined the actual number of leukocytes per ml of blood using a conventional hemacytometer. Then we prepared a smear from the blood, stained the smear with the Giemsa staining solution, and calculated the leukocyte differential (percentage of each type of leukocytes) using a conventional light microscope. This was done in order to determine the need for further dilution of the blood to be assayed. Thus, for the blood containing about 4,000 or more leukocytes per ml and about 20 or 30 percent lymphocytes, sufficient dilutions ranged from 1:10 to 1:50. For blood with a diminished content of leukocytes, especially lymphocytes (i.e., from patients with supressed immunity) dilutions ranged from 1:2 to 1:10. We then diluted the blocd with the culture medium RPMI-1640 (Pharemacia:, Piscataway, NJ) supplemented with 25mM Hepes buffer, human a-gamma serum (1.25 mg/ml protein) and antibiotics (100 U/ml penicillin and 100 U/ml streptomycin). Aliquots of 100 ul of diluted blood were then distributed in wells of a 96 well tissue culture plate. We then added 50 ul of a mitogen, and 50 ul of neuraminidase to each well, and placed the culture plate into a humidified 37° incubator in the presence of 5% $CO_2$.

Any one of the following mitogens can be used for the assay:

1. SEA, diluted in medium from 40 to 80 ng/ml (final concentration from 10 to 20 ng/ml);

2. OKT3 monoclonal antibody diluted in medium from 40 to 80 ng/ml (final dilutions from 10 to 20 ng/ml):

3. ConA diluted in medium 40 ug/ml (final dilution 2.0 ug/ml); or

4. PHAL diluted in medium 20 ug/ml (final dilution 10.0 ug/ml).

Vibrio cholerae neuraminidase (Calbiochem, La Jolla CA) was added at the final concentrations from 0.1 to 0.25 u/ml. As controls, we substituted the mitrogen or neuraminidase with an appropriate volume (50 ul) of the medium. Also, as an additional control, we added 2mCi of radioactive thymidine to some experimental and control wells, 72 hours after commencement of the assay. These cultures were then harvested with a conventional harvesting machine, 18 hours after pulsing with thymydine. We measured thymydine incorporation into mitrogen-stimulated and control cells with a scintillation counter, and expressed the results in cpm.

To assess the expression of autologous E-rosettes in mitogen-stimulated and control cultures, the cells were harvested daily 48, 72, and 96 hours after stimulation. One hour before harvesting, we added Acridine organe, a fluorescent dye, to each well at the final concentration of 2.0 ug/ml. We then harvested each culture to be assayed separately, using a Pasteur pipette. We mounted the cultures on a micro slide or on a hemocytometer, and then examined them with a fluorescent microscope equipped with phase contrast optics. While examining our preparations, we applied a combination of incident (exciting) and transmittent lights. Such a combined illumination enabled us to simultaneously visualize fluorescent leukocytes and non-fluorescent cells, including red blood cells. We were able to determine that red blood cells which formed rosettes around fluorescent lymphocytes, as well as red blood cells which are involved in leukocyte aggregates, are clearly discernable from numerous randomly distributed red blood cells. Because the reticle of the hemacytometer containing whole blood culture was clearly visible, we were able to count rosette-forming lymphocytes, and leukocyte aggregates involving red blood cells.

Our examination demonstrated that in whole blood cultures stimulated with non-erythroagglutinating mitogens in the presence of neuraminidase, both giant autologous E-rosettes and RBC-leukocyte aggregates (largely lymphocyte- monocyte aggregates) involving RBC were formed. Both the giant rosettes and the RBC-leukocyte aggregates (or rosette-like patterns) were stable at 37°. Both were absent in the control cultures stimulated with the same mitogens without neuraminidase; such cultures did show an increased incorporation of a radioactive thymidine. As is shown in Table 1, neither E-rosettes nor combined erythrocyte-leukocyte aggregates were found in non-stimulated whole blood cultures:

## Table 1

BLASTOGENIC RESPONSE, EXPRESSION OF STABLE AUTOLOGOUS
E-ROSETTES AND MIXED RBC-LEUKOCYTE AGGREGATES, AND
PRODUCTION OF GAMMA-INTERFERON IN WHOLE BLOOD CULTURES
INCUDED BY NON-ERYTHROAGGLUTINATING MITOGENS AND
INFLUENCED BY NEURAMINIDASE

| NASE[a] 0.02 U/ml | MITOGENS AND RESPONSE INDICATORS | | | | | | | | | | | |
|---|---|---|---|---|---|---|---|---|---|---|---|---|
| | PHAL 10 ug/ml | | | | SEA 20ug/ml | | | | CON A 10ug/ml | | | |
| | CPM[b] | IFN[c] | SAR[d] | MA[e] | CPM | IFN | SAR | MA | CPM | IFN | SAR | MA |
| – | 30.0 | 300 | 0 | 0 | 7.4 | 90 | 0 | 0 | 13.8 | 190 | 0 | 0 |
| + | 32.0 | 351 | 80 | 93 | 9.3 | 98 | 70 | 90 | 18.1 | 199 | 80 | 100 |

a) Neuraminidase,
b) blastogenic response expressed in counts per min. x $10^{-3}$
c) Units/ml gamma interferon
d) per cent SAR-forming lymphocytes per total single lymphocytes
e) per cent mixed RBC-leukocyte aggregates per total leukocyte aggregates

Our assays of whole blood cultures taken from patients with a decreased number of leukocytes and the decreased number of lymphocytes (as a result of immunosuppressive treatment) showed that expression of stable autologous rosettes and combined erythrocyte-leukocyte aggregates is decreased or even absent.

This was associated with the decrease in the incorporation of radioactive thymidine by the mitogen-stimulated cells indicating that the appearance of stable autologous E-rosettes and rosette-like patterns is relevant to the activation of T lymphocytes (Table 2).

## Table 2

EXPRESSION OF STABLE AUTOLOGOUS ROSETTES (SAR) AND THYMYDINE INCORPORATION (CPM) IN CULTURES OF WHOLE BLOOD TAKEN FROM THE PATIENTS TREATED WITH THE OKT3 MONOCLONAL ANTIBODY FOLLOWING KIDNEY TRANSPLANTATION

| Neura-mini-dase | SAR expression and thymidine incorporation (cpm) upon stimulation with*: | | | | | | | | | |
|---|---|---|---|---|---|---|---|---|---|---|
| | None | | Con A | | PHAL | | OKT3 | | SEA | |
| (U/ml) | cpm | SAR | cpm | SAR | cpm | SAR | cpm | SAR | cpm | SAR |
| – | 51 | – | 106 | – | 382 | – | 88 | – | 1348 | – |
| 0.02 | 92 | – | 146 | – | 2282 | – | 98 | – | 1159 | +/– ** |

* The same concentrations of Con A, PHAL, and SEA as shown in Table 1; 10 ng/ml OKT3 was used

** Occasional E-rosettes and rosette-like patterns were found in SEA-stimulated cultures.

Our observations demonstrate that the presence of neuraminidase in mitogen-stimulated cultures does not interfere with the T cell activation and E-rosetting, but rather enhances T cell blastogenic response. This observation is consistent with reports in the literature [J. Knop, Immunobiology, 157, pp. 474-85, (1980)]. Unlike neuraminidase, AET interferes with both E-rosetting and T cell activation when AET, instead of neuraminidase, is added directly to the leukocyte culture.

EXAMPLE II

Assay Using Isolated Leukocytes

We obtained a heparinized whole blood, and saved a small portion (about 1 ml) of the blood as a source of red blood cells. A 5% suspension of washed red blood cells was prepared from this portion. We isolated leukocytes from the rest of the blood sample using the Ficoll-hypaque procedure or by lysing red blood cells with a 0.83% ammonium chloride solution. We then adjusted the leukocyte suspension to a concentration of $1 \times 10^6$ cells/ml, prepared a smear from a drop of this suspension, and stained it with Giemsa stain solution to calculate the leukocyte differential. This was the first step of the assay. We then distributed aliquots of 50 ul of the leukocyte suspension in wells of a 96 well tissue culture plate, and aliquots of 50 ul of a red blood cell suspension, so as to obtain the optimal ratio of 20 red blood cells to one leukocyte. Then 50 ul of a mitogen, and 50 ml of neuraminidase solutions were added to each well. This was the second step of the assay. The same mitogens and neuraminidase solution were used in this assay as set forth in Example I, above. We incubated culture plates, and examined the stimulated and control cultures.

EXAMPLE III

Assay Using Isolated Leukocytes And Neuraminidase Pretreated Erythrocytes

We prepared a 5% red blood cell suspension, and a suspension of isolated leukocytes $(1 \times 10^6)$ cells/ml as set forth in Example II, above. We then distributed 50 ul aliquots of the leukocyte suspension into wells of a 96 well culture plate. This was the first step of the assay. We then incubated a 5% suspension of red blood cells with 50 U/ml neuraminidase for 30 min at 37°C. We washed the erythrocytes with culture medium twice after incubation and before adding them to the leukocyte cultures. Aliquots of 50 ul of a red blood cell suspension were added to each well containing leukocytes. This was the second step of the assay.

Alternatively, we incubated one volume of red blood cell suspension with 4 volumes of 0.143M solution of AET (pH 9.0) for 15 min. at 37°C. Thereafter, we pelleted red blood cells by centrifugation, washed with culture medium 5 times, and added these cells to the leukocyte culture, instead of neuraminidase-treated cells.

Our data indicated, however, that pretreatment with neuraminidase of leukocyte suspension, rather than the red blood cells, is less efficient with respect to the eventual appearance of autologous E-rosettes in mitogen-stimulated leukocyte cultures.

In the third step, we added a mitogen to each well. The same mitogens were used as set forth above in Examples I and II, above. We incubated plates with stimulated and control cultures, and examined them.

EXAMPLE IV

Assay for Antigen-sensitized T-lymphocytes

Freshly-taken blood examined for the white blood cell number and differential, and diluted with a culture medium, as described in the Example I. Aliquots of 100ul of diluted blood are distributed in wells of a 96 well tissue culture plate. Next, aliquots of 50 ul of the antigen under study and 50 ml of neuraminidase should be added to each well. Because many tissue, bacterial, viral, protozoal antigens are not available through commercial sources, it may be necessary to apply several concentrations of the antigen under study in order to determine its optimal concentration and to standardize the assay conditions.

In control wells, a non-erythroagglutinating mitogen, such as PHAL substitutes for the antigen in order to determine the viability and general responsiveness of T lymphocytes under assessment. Antigen- and mitogen-exposed cultures are incubated for 4 - 5 days at 37° and 5% CO, and then examined for the presence of the mixed RBC-leukocyte aggregates and stable E-rosettes. The optimal time of incubation before examination must be determined for each individual antigen under study. T cell stimulation with antigens may take more time than those with mitogen.

This assay may also be performed using procedures described in Examples II and III.

The following table summarizes the results of our experiments using whole blood (as described in Example I, above) and separated leukocytes (Example II); also presented therein are our data concerning Rickettsial antigens (see Example IV):

## Table 3

BLASTOGENIC RESPONSE TO ANTIGENS OF RICKETTSIA PROWAZEKII AND
RICKETTSIA TYPHI, INTERFERON PRODUCTION, AND FORMATION OF MIXED
RBC-LEUKOCYTE AGGREGATES AND STABLE AUTOLOGOUS E-ROSETTES IN
CULTURE OF WHOLE BLOOD AND SEPARATED LEUKOCYTES

| ANTIGENS MITOGENS | RESPONSE INDICATORS | DONORS AND CULTURES UNDER EVALUATION | | | |
|---|---|---|---|---|---|
| | | DONOR A (seropositive) | | DONOR B (seronegative) | |
| | | CULTURE OF WHOLE BLOOD 1:20 | SEPARATED LEUKOCYTES + AUTOL. RBC | CULTURE OF WHOLE BLOOD 1:20 | SEPARATED LEUKOCYTES + AUTOL. RBC |
| NONE | CPM[a] | 32 | 195 | 37 | 217 |
| | MA[b] | 0 | 0 | 0 | 0 |
| | SAR[c] | 0 | 0 | 0 | 0 |
| | IFN[d] | 0 | 0 | 0 | 0 |
| PHAL 10ug/ml | CPM | 8102 | 21856 | 3894 | 14954 |
| | MA | 100 | 80 | 100 | 73 |
| | SAR | 79 | 10 | 75 | 4 |
| | IFN | 652 | 1271 | 118 | 244 |
| R. thyphi W 10ug/ml | CPM | 213 | 4317 | 31 | 151 |
| | MA | ++[e] | ++ | 0 | 0 |
| | SAR | 0 | + | 0 | 0 |
| | IFN | 176 | 652 | 0 | 0 |
| R. prow. B 10ug/ml | CPM | 430 | 6411 | 40 | 189 |
| | MA | ++ | ++ | 0 | 0 |
| | SAR | 0 | + | 0 | 0 |
| | IFN | 176 | 652 | 0 | 0 |
| LPS E. coli | CPM | 98 | 352 | 30 | 70 |
| | MA | ++ | ++ | 0 | 0 |
| | SAR[e] | 0 | 0 | 0 | 0 |
| | IFN | 127 | ND | 0 | 0 |

a – Blastogenic response expressed as a thymidine incorporation (Counts per min.)
b – Per cent of mixed RBC-leukocyte aggregates per total leukocyte aggregates
c – Per cent of solitary stable E-rosettes per total solitary (single) lymphocytes
d – Units gamma IFN per ml
e – Attachment of RBC to lymphocytes was found only with leukocyte aggregates(MA)

One of donors (A) presented in Table 3 had a history of infection caused by R. prowazekii, and had antibodies against typhus group rickettsiae. Donor B was seronegative to these Rickettsiae. Blood samples from each donor were separated into two parts. One part was diluted 1:20, and used for cultures of whole blood. The second part was used as a source of leukocytes (separated by a Ficoll-hippague procedure) and autologous RBC. Suspensions of $1 \times 10^6$/ml of separated leukocytes were mixed with autologous RBC at the ratio 20 RBC per leukocyte. Aliquots of diluted whole blood and RBC-leukocyte mixture were exposed to neuraminidase, and to either phytohemagglutinin L (PHAL) or one of bacterial antigens, and maintained as cell cultures for five days. Control cultures were exposed only to neuraminidase, without the addition of antigen or mitogen. Some of cultures were then tested for incorporation of radioactive thymidine; others were assayed for interferon-gamma production, and for the presence of stable autologous E-rosettes and mixed RBC-leukocyte aggregates.

Our results demonstrate that: (a) there is no SAR or MA in control (non-stimulated) cultures despite the presence of neuraminidase; (b) in the presence of neuraminidase, polyclonal mitogens, such as PHAL, induce formation of SAR and MA in blood cell cutures from both seropositive and seronegative individuals; (c) unkike polyclonal mitogens, bacterial antigens, such as anti gens of typhus group Rickettsiae, or LPS of E. coli, induce SAR and MA only in cultures from a seropositive donor; (d) upon stimulation with a polyclonal mitogen, such as PHAL, both MA and SAR appear in the whole blood culture simultaneously, which may be expressed as a per cent of SAR-forming-lymphocytes (SAR) per total lymphocytes, and as a per cent of mixed RBC-leukocyte aggregates (MA) per total leukocyte aggregates; (e) in cultures of separated leukocytes, the frequency of MA and SAR seems to be lessened and appears more variable than in whole blood cultures; this appears to be a result of the fragility of MA and (especially) SAR in cultures of separated leukocytes. This fragility may result in technique-dependent variability in the numbers of MA and SAR during microscopical examination. In whole blood cultures, both SAR and MA are stable, and do not undergo technique-dependent alterations; and (f) unlike polyclonal mitogens, bacterial antigens largely induce MA. This confirms that the mitogen-induced T cell activation requires physical contacts between T cells, monocytes, and the antigen. The number of MA and SAR induced by antigens is comparatively low, so this phenomenon should be expressed in semiquantitative indexes (such as +) rather than in figures.

## Claims

1. A method for detecting the presence of activated T-lymphocytes in whole blood comprising the step of incubating whole blood with a mixture of neuraminidase and non-erythroagglutinating mitogen so that T-lymphocytes present form stable E-rosettes.

2. A method for detecting the presence of activated T-lymphocytes in a mixture of isolated leukocytes and autologous erythrocytes comprising the step of incubating the mixture with a mixture of neuraminidase and non-erythroagglutinating mitogen so that T-lymphocytes present form stable E-rosettes.

3. A method for detecting the presence of activated T-lymphocytes in a mixture of isolated leukocytes, and autologous erythrocytes which have been pretreated with neuraminidase, comprising the step of incubating the mixture with a non-erythroagglutinating mitogen so that T-lymphocytes present form stable E-rosettes.

4. A method for detecting the presence of activated T-lymphocytes in a mixture of isolated leukocytes, and autologous erythrocytes which have been pretreated with AET, comprising the step of incubating the mixture with a non-erythroagglutinating mitogen so that T-lymphocytes present form stable E-rosettes.

5. A method acording to any of claims 1 to 4, wherein the mixture is incubated for 48 to 96 hours at 37°C.

6. A method according to any of claims 1 to 5, wherein the mitogen is selected from Staphylococcal enteroxin A, OKT 3 monoclonal antibody, Concanavalin A (Con A), and Phytohemagglutinin L (PHAL).

7. A method for detecting the presence of pre-sensitized T-lymphocytes in whole blood comprising the step of incubating whole blood with a mixture of neuraminidase and an antigen so that T-lymphocytes present form stable E-rosettes.

8. A method for detecting the presence of pre-sensitized T-lymphocytes in a mixture of isolated leukocytes and autologous erythrocytes comprising the step of incubating the mixture with a mixture of neuraminidase and an antigen so that T-lymphocytes present form stable E-rosettes.

9. A method for detecting the presence of pre-sensitized T-lymphocytes in a mixture of isolated leukocytes, and autologous erythrocytes which have been pretreated with neuraminidase, comprising the step of incubating the mixture with an antigen so that T-lymphocytes present form stable E-rosettes.

10. A method for detecting the presence of pre- sensitized T-lymphocytes in a mixture of isolated leukocytes, and autologous erythrocytes which have been pre-treated with AET, comprising the step of incubating the mixture with an antigen so that T-lymphocytes present form stable E-rosettes.

11. A method according to any of claims 7 to 10, wherein the mixture is incubated for 96 to 120 hours at 37°C.

12. A method according to any of claims 7 to 11, wherein the antigen is selected from pathogenic viral, rickettsial, bacterial, and protozoal antigens.

13. A kit comprising a mixture of neuraminidase and a non-erythroagglutinating mitogen for use in detecting the presence of activated T-lymphocytes.

14. A kit comprising a panel of mixtures of neuraminidase and one of a viral, rickettsial, protozoal or bacterial antigen for use in detecting the presence of T-lymphocytes previously sensitized by the respective antigen.